# EUROPÄISCHE PATENTSCHRIFT

(11) **EP 2 227 604 B1**
(45) Veröffentlichungstag und Bekanntmachung des Hinweises auf die Patenterteilung: **28.09.2016**
(21) Anmeldenummer: 08803122.4
(22) Anmeldetag: 21.08.2008
(51) Int. Cl.: E03D 9/00, E03D 9/03, A61L 9/05, A61L 9/12

(54) **ABGABEVORRICHTUNG ZUR ABGABE WENIGSTENS EINES WIRKSTOFFFLUIDS IN DAS SPÜLWASSER EINES TOILETTENBECKENS SOWIE ZUR BEDUFTUNG DER UMGEBUNG**
DISPENSER DEVICE FOR DISPENSING AT LEAST ONE ACTIVE AGENT FLUID INTO THE RINSING WATER OF A TOILET BOWL AND FOR AIR FRESHENING OF THE ENVIRONMENT
DISPOSITIF DISTRIBUTEUR POUR DISTRIBUER AU MOINS UN FLUIDE ACTIF DANS L'EAU DE CHASSE D'UNE CUVETTE DE WC ET POUR PARFUMER L'ENVIRONNEMENT

(30) Priorität: 08.01.2008 DE 102008003359
(43) Veröffentlichungstag der Anmeldung: 15.09.2010
(73) Patentinhaber: Henkel AG & Co. KGaA, 40589 Düsseldorf (DE)
(72) Erfinder: BUTTER-JENTSCH, Ralph, 40764 Langenfeld (DE); PESSEL, Frank, 40215 Düsseldorf (DE); MÜHLHAUSEN, Hans-Georg, 40597 Düsseldorf (DE)
(86) Internationale Anmeldenummer: PCT/EP2008/060922
(87) Internationale Veröffentlichungsnummer: WO 2009/086947

(56) Entgegenhaltungen:
- EP-A- 1 469 132
- WO-A-03/027405

## Beschreibung

Die Erfindung betrifft eine Abgabevorrichtung zur Abgabe wenigstens eines Wirkstofffluids in das Spülwasser eines Toilettenbeckens sowie zur Beduftung der Umgebung bei dem Spülwasser zur Erzeugung einer spülwassergesteuerten Erhöhung der Duftstofffreisetzung auf eine membranüberdeckte Öffnung einer Duftstoff enthaltenden Funktionskammer geleitet wird.

### Stand der Technik

Aus dem Stand der Technik ist eine Vielzahl von unterschiedlichen Wirkstoffabgabesystemen für Toilettenbecken bekannt.

Am weitesten verbreitet sind Wirkstoffabgabesysteme, welche mit einem Halter am Rand eines Toilettenbeckens befestigt und innenseitig in der Toilette im Spülwasserstrom angeordnet werden. Derartige Wirkstoffabgabesysteme werden gewöhnlich auch als WC-Spüler bezeichnet.

Es existieren Wirkstoffabgabesysteme für Wirkstoffzubereitungen bei denen die Wirkstoffe aus einer festen Matrix herauslösbar sind. Derartige, feste Wirkstoffzubereitungen werden auch als sog. Rim-Blocks bezeichnet. Bekannt sind ferner Wirkstoffzubereitungen in gelförmiger und flüssiger Form.

Allen dieser Wirkstoffzubereitungen ist üblicherweise gemein, dass sie einen oder mehrere Duftstoffe beinhalten.

Bekannt sind zunächst Abgabevorrichtungen für eine einzelne Wirkstoffzubereitung. Die Zubereitung befindet sich üblicherweise in einem Halter fest angeordneten oder auswechselbar eingesetzten Vorratsbehälter. Mit einem am Halter ausgebildeten Bügel wird das Wirkstoffabgabesystem am Rand der Toilette befestigt, wobei der Vorratsbehälter oder dafür vorgesehene Abgabeeinrichtungen, beispielsweise Abspülplatten, auf der Toiletteninnenseite unmittelbar unterhalb des Toilettenrandes anliegen, so dass beim Spülwasseraustritt aus dem innenseitigen Toilettenrand der Vorratsbehälter oder die entsprechenden Abgabevorrichtungen vom Spülwasser überströmt werden. Derartige Abgabevorrichtungen sind beispielsweise aus EP1334239 bekannt.

Ein wesentlicher Nachteil derartiger Wirkstoffabgabesysteme ist es, dass eine Beduftung des Toilettenbeckens bzw. der Umgebung des Toilettenbeckens im Wesentlichen an den Spülvorgang der Toilette gekoppelt ist, da derartige Wirkstoffabgabesysteme so ausgebildet sind, dass üblicherweise nur dann ein Parfum enthaltener Wirkstoff aus den eingangs genannten Wirkstoffabgabesystemen freigesetzt wird, wenn das Wirkstoffabgabesystem von Spülwasser überströmt wird.

So ist der Dufteindruck intervallartig und regelmäßig von nur kurzer Dauer. Insbesondere wird keine Beduftung vor oder länger andauernd nach Benutzung einer Toilette durch die Wirkstoffabgabesysteme der eingangs geschilderten Art bewirkt.

Dieser Nachteil führte zur Entwicklung von WC-Abgabesystemen der eingangs geschilderten Art, die eine permanente Abgabe eines Duftstoffs in die Umgebung eines Toilettenbeckens erlauben.

Aus WO2005/093176 ist beispielsweise ein WC-Körbchen bekannt, in dessen Gehäuse ein gelförmiger, Duftstoff enthaltener Block fest eingegossen ist. Nachteilig an dieser Lösung ist, dass der gelförmige Block derart angeordnet ist, dass er von Spülwasser überströmt und somit allmälig aufgelöst wird, wodurch nach und nach ein äußerst unanehmlicher optischer Eindruck entsteht.

Dieses Problem wird beispielsweise durch ein WC-Wirkstoffabgabesystem gemäß WO2006/005410 vermieden. Bei dem aus der vorgenannten Offenbarung bekannten WC-Körbchen, sind Duftstoff enthaltende Kammern am Halter der Abgabevorrichtung angeordnet. Durch das Einschließen der Duftstoffzubereitung in einer Kammer, wird eine unbeabsichtigte Freisetzung der Zubereitung durch spülwasserbedingte Auflösung verhindert. Ferner wird es durch die Verwendung einer Kammer möglich Flüssigkeiten, welche mit höheren Konzentrationen an Duftstoff beladbar sind, als Duftstoffträger einzusetzen.

In diesem Zusammenhang sind auch auf permeable Membranen basierte Duftstoffabgabesysteme bekannt. Dabei ist die Öffnung des Behälters, der üblicherweise einen Duftstoff in einer flüssigen Matrix enthält, vollständig mit einer für Duftstoffe permeablen Membran überdeckt, so dass die flüssige Trägermatrix nicht aus dem Behälter austreten kann, jedoch Duftstoffe durch die Membran an die Umgebung abgegeben werden.

Derartige Duftabgabesysteme realisieren zwar eine permanente Beduftung der Umgebung des WC-Spülers, jedoch ist deren Duftfreisetzungscharakteristik im Wesentlichen gleichförmig, d.h. unabhängig vom Spülwasserstrom und somit unabhängig von der Benutzung der Toilette.

Es ist jedoch wünschenswert, eine gewisse Permanentbeduftung der Umgebung des WC-Spülers bereitzustellen, jedoch gleichzeitig eine verstärkte Duftstoffabgabe unmittelbar beim Spülen sowie einer erhöhten Duftstoffabgabe in einem Zeitintervall unmittelbar nach dem Spülvorgang zu realisieren, da zu diesen Zeiten die Belastung der Umgebungsluft mit einer erhöhten Konzentration an Schlechtgerüchen potentiell am höchsten ist und somit eine erhöhte Duftstofffreisetzung als besonders angenehm empfunden wird.

### Aufgabe der Erfindung

Aufgabe der Erfindung ist es daher eine Abgabevorrichtung zur Abgabe von Wirkstofffluiden in das Spülwasser eines Toilettenbeckens sowie zur Beduftung der Umgebung der Abgabevorrichtung mit einem optimierten Duftfreisetzungsprofil bereitzustellen.

Diese Aufgabe wird durch eine Abgabevorrichtung mit den Merkmalen des Anspruchs 1 gelöst.

Mittels einer gezielten Überströmung der permeablen Membran der Funktionskammer und Benetzung der Membran mit Spülwasser, können in überraschender Weise verschiedene vorteilhafte Effekte erzielt werden.

Durch die Benetzung der Membran mit in der Regel kaltem Spülwasser, wird durch die Verdampfung des Wassers von der benetzten Membranoberfläche bei üblicherweise in einer Toilette vorherrschenden Raumtemperatur zwischen 16-24°C eine Konvektion über der Oberfläche der Membran erzeugt, die eine erhöhte Freisetzung von Duftstoff aus der Funktionskammer bewirkt. Da diese Konvektion üblicherweise erst mit einiger Verzögerung nach der Anspülung und Benetzung der Membran einsetzt, kann hierdurch neben einer gleichförmigen Freisetzung von Duftstoff aus dem Membransystem auch eine erhöhte und länger andauernde erhöhte Freisetzung unmittelbar nach Betätigung der Toilettenspülung realisiert werden. Dies ist insbesondere zur Bekämpfung von Schlechtgerüchen unmittelbar nach Benutzung einer Toilette von Vorteil, zu der hierdurch eine erhöhte Menge an Duftstoff freigesetzt wird.

### Funktionskammer

Eine Funktionskammer im Sinne dieser Anmeldung bezeichnet einen an der Abgabevorrichtung angeordneten Raum, der dazu geeignet ist, ein Duftstoff emittierendes Trägermaterial im Wesentlichen vollständig zu umhüllen und/oder zusammenzuhalten.

Die Funktionskammer kann insbesondere als jegliche Art von Behälter, Ampulle, Dose, Beutel, Flasche, Kartusche, Kapsel, Aufwickelhülse, Rollenkern, Ballon, Flakon, Röhrchen, Tiegel, Kammer, Blister, Korb oder Geflecht ausgebildet sein. Im Sinne dieser Anmeldung umfasst die Funktionskammer auch einen aus einer festen oder gelförmigen Zubereitung geformten Körper.

Die Funktionskammer kann aus einem oder mehreren voneinander getrennten oder beabstandeten Räumen bestehen. Dies ist insbesondere zur Bevorratung von miteinander nicht lagerstabilen Zubereitungen von Vorteil.

Die Funktionskammer kann auch lösbar an der Abgabevorrichtung fixiert sein, insbesondere durch Form-, Kraft,- und/oder Stoffschluss.

Die Funktionskammer und die Vorratsbehälter können insbesondere so ausgebildet sein und/oder zusätzliche Verbindungsmittel umfassen, so dass die Funktionskammer und die Vorratsbehälter formschlüssig beispielsweise durch einen Schnappverschluss, Rastverschluss, Prellverschluss, Schraubverschluss, Bajonettverschluss, Klemmverschluss, Quetschverschluss oder Druckknopfverschluss aneinander lösbar oder fest fixierbar sind.

In einer weiteren Ausgestaltungsform der Erfindung kann die Funktionskammer auch stoffschlüssig an einem Vorratsbehälter angeordnet sein. Die stoffschlüssige Verbindung ist insbesondere in derart ausgebildet, dass ein wiederholtes Lösen und Haften der Funktionskammer auf bzw. an dem Vorratsbehälter ermöglicht wird. Die stoffschlüssige Verbindung kann beispielsweise aus der Gruppe der Klebeverbindungen, Schweißverbindungen oder Siegelverbindungen ausgewählt sein.

Ferner ist es möglich die lösbare Verbindung zwischen der Funktionskammer und einem Vorratsbehälter durch Kraftschluss herzustellen. Dies kann beispielsweise durch eine Pressverbindung, Reibverbindung, Klemmverbindung, Quetschverbindung, Schrumpfverbindung, Keilverbindung, Schraubverbindung, Stiftverbindung oder dergleichen realisiert sein.

Selbstverständlich kann die lösbare Verbindung zwischen der Funktionskammer und einem Vorratsbehälter auch durch eine beliebige Kombination der vorgenannten Verbindungsarten ausgebildet sein.

Bevorzugter Weise weist die Funktionskammer ein Volumen zwischen 5 ml und 100 ml, insbesondere zwischen 10 ml und 40 ml, auf.

Gemäß dieser Erfindung weist die Trägermaterial enthaltende Funktionskammer wenigstens eine mit einer permeablen Membran versehenen Öffnung zur Freisetzung der in ihr bevorrateten Duftstoffzubereitung auf.

Zur Freisetzung der Aktivsubstanzen aus der Kammer in die Umgebung weist die Kammer mindestens eine Öffnung auf. Es können auch mehrere mit einer oder mehreren Membranen verschlossene Öffnungen in der Kammer ausgebildet sein. Die Öffnung und die Membran bevorzugt so ausgestaltet, dass das Trägermaterial die Öffnung nicht passieren kann. Die Funktionskammer ist in derart ausgebildet, dass die Membran von Spülwasser über-, bzw. angeströmt werden kann.

Durch die Dimensionierung der Öffnungen und Konfiguration der Membran ist es möglich, die konstante Duftstofffreisetzungsrate aus der Funktionskammer einzustellen.

Das Trägermaterial ist vorzugsweise eine Flüssigkeit.

Des Weiteren ist es bevorzugt, dass die Funktionskammer zwischen zwei Vorratsbehältern angeordnet ist. Hierdurch ragt die Funktionskammer nicht über Rand des Halters hinaus in das Toilettenbecken hinein und kann gemeinsam mit dem Halter diskret am oder unter den Rand des Toilettenbeckens positioniert werden. Ferner ist durch die Anordnung der Funktionskammer zwischen den Vorratsbehältern ein gewisser Klemmeffekt erreichbar, durch den die Funktionskammer sicher zwischen den Vorratsbehältern fixierbar ist.

Durch die Ausbildung der Funktionskammer als Prismatoid und entsprechend ausgeformten Vorratsbehältern ist diese Klemmwirkung bzw. Fixierung weiter optimierbar. Insbesondere kann die Funktionskammer beispielsweise pyramidenförmig, keilförmig, kuppelförmig, kegelstumpfförmig oder prismenförmig ausgebildet sein.

Durch die entsprechend korrespondierende Formgestaltung der Funktionskammer und Vorratsbehälter wird die Funktionskammer beim Einsetzen bei der Konfektionierung des Produktes oder durch den Benutzer trichterartig in die in der Nachfülleinheit vorgesehene Position geführt, wodurch Fehlbedienungen beim Einsetzen vermieden werden.

### Trägermaterial

In der Funktionskammer ist wenigstens ein Duftstoff emittierendes Trägermaterial angeordnet.

Die Funktionskammer kann eine oder mehrere Aktivsubstanzen beinhaltende Zubereitungen in fester und/oder flüssiger und/oder gelartiger und/oder gasförmiger Form umfassen, welche an bzw. in Trägermaterialien gebunden oder gelöst sind.

Die Trägermaterialien können einen festen und/oder flüssigen und/oder gelartigen und/oder gasförmigen Aggregatszustand aufweisen. In einer Ausgestaltungsform der Erfindung ist die Funktionskammer selbst der Träger der Aktivsubstanzen.

Insbesondere kann ein Trägermaterial auch ein poröser Körper sein, wie etwa ein Schwamm. Auch ist es denkbar, dass das Trägermaterial aus Zellulose besteht, wie etwa einem Watte-Pad.

Gemäß einer bevorzugten Ausführungsform der Erfindung, sind die wirkaktiven Substanzen an oder in einem polymeren Trägermaterial gebunden. Besonders bevorzugt werden Duftstoffe in oder an einem polymeren Trägermaterial gebunden.

Weitere Trägermaterialen können beispielsweise auf der Basis von Cellulose, porösen Materialen, wie beispielsweise Schwämmen, abspülbaren oder nicht abspülbaren Gelen, Fluiden, wasserlöslichen Folien oder dergleichen ausgewählt sein.

Die Trägermaterialien können auch optische und/oder akustische Signale erzeugen, beispielsweise durch Fluoreszenz, Phosphoreszenz oder Aufplatzen von zellulären Strukturen. Hierdurch lassen sich beispielsweise optische und/oder akustische Verbrauchsanzeigen realisieren.

### Vorratsbehälter

Die erfindungsgemäße Abgabevorrichtung umfasst wenigstens einen Vorratsbehälter zur Aufnahme einer in das Spülwasser eines Toilettenbeckens abgebbaren Zubereitung.

In einer bevorzugten Ausführungsform der Erfindung ist mindestens ein weiterer Vorratsbehälter vorgesehen, der eine Zubereitung beinhaltet, die von den Zubereitungen der anderen Vorratsbehälter verschieden ist. Dies ist insbesondere vorteilhaft, wenn Zubereitungen verwendet werden sollen, die üblicherweise nicht gemeinsam lagerfähig sind, wie beispielsweise Bleichen und Duftstoffe.

Die Vorratsbehälter können einstückig oder mehrstückig ausgeformt sein. Die einstückige Ausformung von beispielsweise zwei Vorratsbehältern kann durch ein geeignetes Blasformverfahren realisiert sein, wobei die Vorratsbehälter unter Ausbildung eines gemeinsamen Verbindungsstegs einstückig miteinander verbunden sind. Bei einer zweistückigen Ausgestaltung können die beiden voneinander separierbaren Vorratsbehälter beispielsweise über ein entsprechendes Adapterelement bzw. Verbindungselement gegeneinander fixiert sein.

### Duftstoff

Ein Duftstoff im Sinne dieser Anmeldung ist eine olfaktometrisch wahrnehmbare fließfähige, streufähige, gelförmige, gasförmige oder feste Zusammensetzung.

Die Funktionskammer kann auch eine oder mehrere Duftstoffzubereitungen in fester und/oder flüssiger und/oder gelartiger Form beinhalten. Dabei ist es auch denkbar, die entsprechenden Zubereitungen räumlich getrennt voneinander in der Funktionskammer zu bevorraten, um eine ungewollte Vermischung der Zubereitungen zu verhindern.

Die Duftstoffe sind an oder in Trägermaterialen gebunden oder gelöst. Die Trägermaterialien können einen festen und/oder flüssigen und/oder gelartigen Aggregatszustand aufweisen.

Ferner ist es auch möglich, neben den Duftstoffen, Indikatorsubstanzen zur Bestimmung der Wasserhärte, Keimbelastung, Temperatur, Feuchtigkeit usw. in der Funktionskammer zu bevorraten.

Insbesondere kann es nach einer bevorzugten Ausführungsform der Erfindung vorteilhaft sein, eine Abgabe von Aktivstoff in zwei oder mehr Phasen vorzusehen. Dies ist insbesondere dann vorteilhaft, wenn die beiden abzugebenden Phasen nicht gemeinsam lagerstabil sind oder bei der Abgabe von Duftstoff, eine olfaktometrische Anpassung an den abgegebenen Duft vermieden werden soll.

Im Folgenden wird die Erfindung anhand von lediglich Ausführungsbeispiele darstellenden Zeichnungen näher erläutert. Dabei werden auch besonders bevorzugte Ausgestaltungen und besonders bevorzugte Kombinationen von Merkmalen im Einzelnen weiter beschrieben. Es zeigt:
- Fig. 1: Abgabevorrichtung in perspektivischer Sicht auf die dem innenseitigen Rand eines Toilettenbeckens zugewandte Seite
- Fig. 2: Abgabevorrichtung in perspektivischer Sicht auf die Vorderseite
- Fig. 3: Abgabevorrichtung in der Seitensansicht
- Fig. 4: Befestigungselement mit Kanal in perspektivischer Ansicht
- Fig. 5: Nachfülleinheit in Explosionsdarstellung

### Bezugszeichenliste

- 1.: Abgabevorrichtung
- 2.: Halter
- 3a,3b.: Vorratsbehälter
- 4a,4b.: Auslassöffnungen
- 5.: Aufnahme
- 6.: Verteilungselement
- 7.: Funktionskammer
- 8.: Kanal
- 9.: Membran
- 10.: Befestigungselement
- 11.: Öffnungen
- 12.: Öffnungen
- 13.: Verbindungselement
- 14.: Rastverbindung
- 15.: Schaft
- 16.: Wanne
- 17.: Verschluss
- 19.: Toilettenbecken
- 20.: Spülwasseraustritt

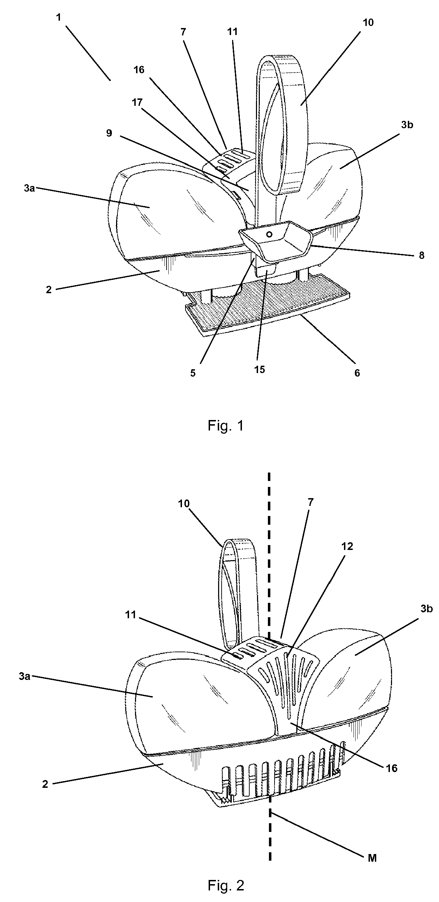

Fig. 1 zeigt eine Ausführungsform der erfindungsgemäßen Abgabevorrichtung 1 in perspektivischer Sicht auf die dem innenseitigen Rand eines Toilettenbeckens im bestimmungsgemäßen Gebrauch zugewandten Seite der Abgabevorrichtung 1.

Die Abgabevorrichtung 1 weist einen Halter 2 auf, in dem ein erster Vorratsbehälter 3a und ein zweiter Vorratsbehälter 3b angeordnet sind. Es ist auch möglich, abweichend von der dargestellten Konfiguration, auch nur einen Vorratsbehälter oder mehr als 2 Vorratsbehälter zur Abgabe darin enthaltener Wirkstofffluide vorzusehen.

Der Halter 2 kann am Rand eines Toilettenbeckens aufgehängt werden. Hierzu ist am Halter 2 ein bügelförmiges Befestigungselement 10 vorgesehen. Selbstverständlich können auch alternative Befestigungsvorrichtungen wie Saugnäpfe oder Klebestreifen als Befestigungselement ausgewählt werden.

Das Befestigungselement 10 weist an seinem unteren Ende einen Schaft 15 auf, der das Befestigungselement 10 über die Aufnahme 5 an der Abgabevorrichtung fixiert, bevorzugt über eine Schnapp-Rast-Verbindung.

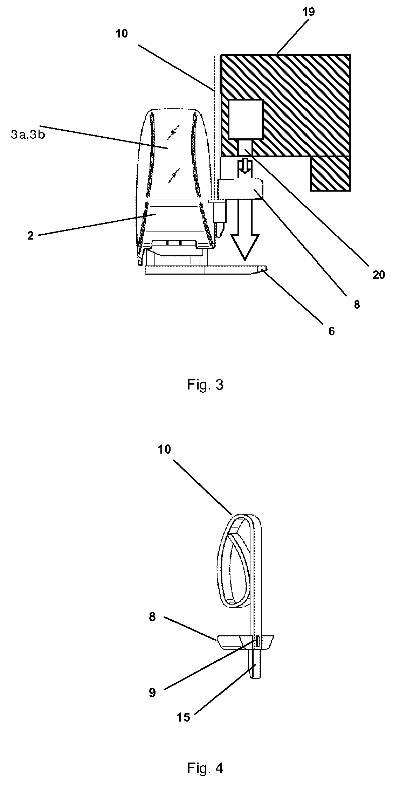

Eine Einzeldarstellung des Befestigungselements 10 kann Fig. 4 entnommen werden. Man erkennt, dass die Öffnung sich durch den Kanal 8 und das Befestigungselement 10 hindurch erstreckt, so dass Spülwasser über den Kanal 8 auf die mit der Membran 9 verschlossene Funktionskammer 7 geleitet wird.

Die Vorratsbehälter 3a,3b weisen jeweils wenigstens eine Auslassöffnung 4a, 4b, die in dieser Ansicht von Fig. 1 nicht sichtbar sind, auf, über die das Wirkstofffluid in die Spülflüssigkeit abgebbar ist, wobei die Vorratsbehälter 3a,3b gegen den Eintritt von Spülflüssigkeit in ihr Inneres geschützt sind und die Auslassöffnung 4a, 4b der Vorratsbehälters 3a,3b so angeordnet sind, dass nur Wirkstofffluid austritt und die Auslassöffnungen 4a,4b des Vorratsbehälters 3a,3b in Gebrauchsstellung bodenseitig angeordnet sind.

Bei jedem Spülvorgang erfolgt die Abgabe einer Teilmenge des Wirkstofffluids aus den Vorratsbehältern 3a,3b in die Spülflüssigkeit über ein am Halter 2 vorgesehenes plattenartiges Verteilungselement 6, das einen beim Spülvorgang von Spülflüssigkeit überströmten Beaufschlagungsbereich aufweist und dass das Innere der Vorratsbehälter 3a,3b über die Auslassöffnungen 4a,4b unter Zwischenanordnung einer ein freies Fließen des Wirkstofffluids verhindernden Anordnung dauernd mit dem Verteilungselement 6 in Verbindung steht.

Zwischen den Vorratsbehältern 3a,3b ist im Halter 2 eine Funktionskammer 7 zur Aufnahme eines Duftstoff emittierenden Trägermaterials vorgesehen.

Auf der dem Spülwasserstrom zugewandten Seite weist die Funktionskammer eine Öffnung auf, die von einer Membran 9 vollständig überbedeckt ist.

Die Funktionskammer 7, die Membran 9 und das Trägermaterial (nicht sichtbar) sind in derart ausgestaltet, dass Spülwasser nicht in die Funktionskammer 7 eintreten kann. Die Funktionskammer 7 und die Membran 9 ist in derart konfiguriert ist, dass sie das Trägermaterial, vorzugsweise eine Flüssigkeit, im Wesentlichen vollständig umschließt wobei das Trägermaterial in der Funktionskammer 7 zurückgehalten wird und Duftstoffe aus dem Trägermaterial in die Umgebung freisetzbar sind. Durch den Kanal 8 wird beim Spülvorgang Spülwasser zu der dem innenseitigen Toilettenrand zugewandten Seite der Funktionskammer 7 geleitet, so dass die Membran 9 von Spülwasser benetzt wird.

Die von der Membran 9 überdeckte Öffnung und die Membran 9 können jede geeignete Gestalt annehmen. Sie kann insbesondere kreisförmig, oval, V-, U- oder schlitzförmig ausgebildet sein.

Die Abgabevorrichtung 1 weist einen Kanal 8 auf, mit dem Spülwasser zur Funktionskammer 7 geleitet wird, so dass die Membranfläche 9 der Funktionskammer 7 bei jedem Auslösen des Spülwasserstroms von Spülwasser angeströmt wird. Im gezeigten Beispiel ist dieser Kanal 8 am Befestigungselement 10 angeordnet. Es ist jedoch auch denkbar, den Kanal 8 an einer beliebig anderen Stelle der Abgabevorrichtung 1 anzuordnen, beispielsweise am Halter 2, an der Funktionskammer 7 oder an einem Vorratsbehälter 3, solange durch den Kanal 8 Spülwasser zur Membran 9 der Funktionskammer 7 geleitet wird.

Ferner kann der Kanal 8 jede geeignete Form annehmen, solange diese geeignet ist, Spülwasser vom Rand der Toilette auf die Membran 9 der Funktionskammer 7 zu leiten. Insbesondere kann der Kanal 8 eine U- oder V-förmige Querschnittsform aufweisen. Es ist jedoch auch denkbar, den Kanal 8 geschlossen, also rohrförmig auszubilden. Des Weiteren kann der Kanal 8 in einer alternativen Ausführung als Strömungsleitplatte ausgestaltet sein, die einen Teil des Spülwasserstroms in Richtung der Funktionskammer 7 lenkt.

Durch die Konfiguration des Kanals 8, der membranbedeckten Öffnung, der Membran 9 sowie der Ausgestaltung und Beladung der Trägermaterialien, lässt sich das Duftstofffreisetzungsprofil des WC-Spülers 1 in weiten Grenzen einstellen.

In Gebrauchstellung der in Fig. 1 gezeigten Abgabevorrichtung liegt der Kanal 8 im Wesentlichen direkt unterhalb des Spülwasseraustritts 20 aus dem Rand des Toilettenbeckens 19, wie dies insbesondere durch die Darstellung in Fig. 3 ersichtlich wird.

Durch die Ausgestaltung des Befestigungselements 10 und des Halters 2 ist gewährleistet, dass nur der Kanal 8 und das plattenartige Verteilungselement 6 im direkten Spülwasserstrom liegen, die Vorratsbehälter 3a,3b sowie die Funktionskammer 7 werden dementsprechend nicht direkt von Spülwasser überströmt. Somit wird verhindert, dass die optisch attraktiven und in der Toilettenschüssel für den Benutzer sichtbaren Bereiche nach mehrmaligem Auslösen des Spülwasserstroms durch mögliche Verschmutzungen des Spülwasserstroms beginnen durch Verschmutzungen, Ablagerungen, Verfärbung oder ähnliches unansehnlich zu werden.

Durch die Konfiguration der Form, Anordnung und Größe der membranbedeckten Öffnungen 11, 12 kann der Benetzungsgrad der Membran 9 eingestellt werden.

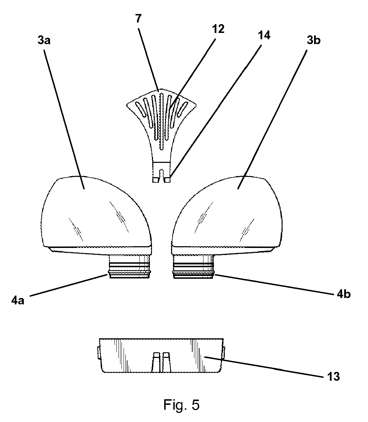

Die Funktionskammer 7 weist - wie der Fig. 2 zu entnehmen ist - auf Ihrer Vorderseite 16 im Wesentlichen vertikal verlaufende schlitzförmige Öffnungen 12 auf. Diese schlitzförmigen Öffnungen 12 liegen nicht im direkten Spülwasserstrom, sodass im Gebrauchszustand kein Spülwasser auf die membranbedeckten Öffnungen 12 der Funktionskammer gelangen kann. Die schlitzförmigen Öffnungen 12 erstrecken sich, wie aus Fig. 2 und Fig. 5 ersichtlich, nicht vollständig bis zum Boden der Funktionskammer 7, sondern enden in einem definierten Abstand oberhalb des Bodens. Durch die schlitzförmigen membranbedeckten Öffnungen 12 können auch Duftstoffe aus der Funktionskammer 7 an die Umgebung abgegeben werden. Wie aus Fig. 2 weiter ersichtlich, können auch membranbedeckte Öffnungen 11 auf der Kopffläche der Kammer angeordnet sein, durch die von den Trägermaterialen emittierten Duftstoffe hindurch an die Umgebung abgegeben werden.

Fig. 5 zeigt eine Explosionsdarstellung einer Nachfülleinheit für den Halter 2 der Abgabevorrichtung 1. Die Nachfülleinheit wird gebildet durch einen ersten Vorratsbehälter 3a und einen zweiten Vorratsbehälter 3b, welche mit ihren bodenseitigen Öffnungen 4a,4b mit dem Verbindungselement 13 gekuppelt werden, so dass ihre relative Lage zueinander festgelegt ist. Die Vorratsbehälter 3a,3b weisen auf den ihnen jeweils zugewandten Seiten eine bogenförmige Kontur auf, die im Zusammengefügten Zustand der Nachfülleinheit einen in etwa V-förmigen Zwischenraum definieren.

Die Funktionskammer 7 weist dieser bogenförmigen Kontur folgende Seitenwände auf, so dass die Seitenwände der Funktionskammer zumindest abschnittsweise auf der Kontur der Vorratsbehälter aufliegen. Die Funktionskammer 7 wird über die Rastverbindung 14 form- oder kraftschlüssig an bzw. in dem Verbindungselement 13 fixiert.

## Patentansprüche

1. Abgabevorrichtung (1) zur Abgabe wenigstens eines Wirkstofffluids in das Spülwasser eines Toilettenbeckens sowie zur Beduftung der Umgebung der Abgabevorrichtung (1) umfassend
▪ einen am Rand des Toilettenbeckens aufhängbaren Halter (2) und
▪ mindestens einen im Halter (2) vorgesehenen Vorratsbehälter (3a,3b) für ein Wirkstofffluid, wobei der Vorratsbehälter (3a,3b) wenigstens eine Auslassöffnung (4a,4b) aufweist, über die das Wirkstofffluid in die Spülflüssigkeit abgebbar ist, wobei
▪ der Vorratsbehälter (3a,3b) gegen den Eintritt von Spülflüssigkeit in sein Inneres geschützt ist und die Auslassöffnung (4a,4b) des Vorratsbehälters so angeordnet ist, dass nur Wirkstofffluid austritt,
▪ dass die Auslassöffnung (4a,4b) des Vorratsbehälters in Gebrauchsstellung bodenseitig angeordnet ist,
▪ dass bei jedem Spülvorgang die Abgabe einer Teilmenge des Wirkstofffluids aus dem Vorratsbehälter in die Spülflüssigkeit erfolgt,
▪ dass am Halter (2) ein plattenartiges Verteilungselement (6) vorgesehen ist, das einen beim Spülvorgang von Spülflüssigkeit überströmten Beaufschlagungsbereich aufweist,
▪ dass das Innere der Vorratsbehälter (3a,3b) über die Auslassöffnung (4a,4b) unter Zwischenanordnung einer ein freies Fließen des Wirkstofffluids verhindernden Anordnung dauernd mit dem Verteilungselement (6) in Verbindung steht,
▪ wobei wenigstens eine Funktionskammer (7) zur Aufnahme eines Duftstoff emittierenden Trägermaterials an der Abgabevorrichtung (1) vorgesehen ist, wobei
▪ die Funktionskammer (7) derart konfiguriert ist, dass sie das Trägermaterial im Wesentlichen vollständig umschließt und
▪ kein Spülwasser in die Funktionskammer (7) eintreten kann wobei
▪ die Funktionskammer (7) wenigstens eine von einer Duftstoff-permeablen Membran (9) verschlossenen Öffnung aufweist, so dass
▪ das Trägermaterial in der Funktionskammer (7) zurückgehalten wird und Duftstoffe vom Trägermaterial aus der Funktionskammer (7) in die Umgebung freisetzbar sind, **dadurch gekennzeichnet, dass**
▪ die Abgabevorrichtung (1) wenigstens ein Mittel (8) aufweist, mit dem Spülwasser auf die Membran (9) geleitet wird, so dass die Membran (9) bei jedem Auslösen des Spülwasserstroms von Spülwasser überströmt und benetzt wird.

2. Abgabevorrichtung nach Anspruch 1, **dadurch gekennzeichnet, dass** die Funktionskammer (7) eine zum Mittel (8), mit dem Spülwasser in die Funktionskammer (7) geleitet wird, hin gerichtete membranbedeckte Öffnung (9) aufweist.

3. Abgabevorrichtung nach einem der vorherigen Ansprüche, **dadurch gekennzeichnet, dass** die Funktionskammer (7) kopfseitige, membranbedeckte Öffnungen (11) und/oder Öffnungen (12) in der Mantelfläche aufweist, die einen Austritt Duftstoff aus der Funktionskammer (7) erlauben.

4. Abgabevorrichtung nach einem der vorherigen Ansprüche, **dadurch gekennzeichnet, dass** die Funktionskammer (7) aus einem ersten wannenförmigen Teil (16) und einem zweiten, das wannenförmige Teil (16) verschließenden Teil (17) gebildet ist.

5. Abgabevorrichtung nach einem der vorherigen Ansprüche, **dadurch gekennzeichnet, dass** der Halter (2) und das Befestigungselement (10) in derart konfiguriert sind, dass die Funktionskammer (7) in der Gebrauchstellung der Abgabevorrichtung (1) außerhalb des Spülwasserstroms positioniert ist.

6. Abgabevorrichtung nach einem der vorherigen Ansprüche, **dadurch gekennzeichnet, dass** das Trägermaterial eine Flüssigkeit ist.

7. Abgabevorrichtung nach einem der vorherigen Ansprüche, **dadurch gekennzeichnet, dass** das Trägermaterial einen festen oder gelartigen Aggregatszustand aufweist.

8. Abgabevorrichtung nach einem der vorherigen Ansprüche, **dadurch gekennzeichnet, dass** das Trägermaterial ausgewählt ist aus der Gruppe der Polymere, Cellulosen, porösen Materialen, Schwämmen, abspülbaren oder nicht abspülbaren Gele, wasserlöslichen oder unlöslichen Folien, Alkohole, Öle oder wässrigen Lösungen.

9. Abgabevorrichtung nach einem der vorherigen Ansprüche, **dadurch gekennzeichnet, dass** das Mittel, mit dem Spülwasser in die Funktionskammer (7) geleitet wird, als oben offener, schaufelartiger Kanal (8) ausgebildet ist, der in derart an der Abgabevorrichtung angeordnet ist, dass im bestimmungsgemäßen Gebrauchszustand der Abgabevorrichtung am Toilettenbecken der Kanal (8) unterhalb des Spülwasseraustritts des Toilettenbeckens liegt und sich zumindest ein Teil des Spülwassers beim Austritt aus dem innenseitigen Rand des Toilettenbeckens im Kanal (8) sammelt und von dort auf die Membran (9) der Funktionskammer (7) geleitet wird.

10. Abgabevorrichtung nach Anspruch 9, **dadurch gekennzeichnet, dass** der Kanal (8) oberhalb des plattenförmigen Verteilungselements (6) angeordnet ist.

11. Abgabevorrichtung nach einem der Ansprüche 9 und 10, **dadurch gekennzeichnet, dass** der Kanal (8) und das plattenförmige Verteilungselement (6) zum innenseitigen Rand eines Toilettenbeckens hin gerichtet in derart in das Toilettenbecken hineinragen, dass die Abgabevorrichtung (1) im bestimmungsgemäßen Gebrauchszustand im Toilettenbecken in einer im wesentlichen vertikalen Position bezüglich der Achse (M) festgelegt ist.

12. Abgabevorrichtung nach einem der Ansprüche 9-11, **dadurch gekennzeichnet, dass** der Kanal (8) am Befestigungselement (10) fixiert ist.

13. Abgabevorrichtung nach einem der vorherigen Ansprüche, **dadurch gekennzeichnet, dass** mindestens ein weiterer Vorratsbehälter (3b) für ein Wirkstofffluid im Halter (2) vorgesehen ist, wobei der Vorratsbehälter (3b) eine Auslassöffnung (4b) aufweist, über die das Wirkstofffluid in die Spülflüssigkeit abgebbar ist.

## Claims

1. A dispensing device (1) for dispensing at least one active liquid into the flushing water of a toilet bowl and for fragrancing the area surrounding the dispensing device (1), comprising
• a holder (2) which can be hung on the rim of the toilet bowl and
• at least one storage container (3a, 3b), provided in the holder (2), for an active liquid, the storage container (3a, 3b) comprising at least one outlet opening (4a, 4b) via which the active liquid can be dispensed into the flushing liquid,
• the storage container (3a, 3b) being protected against the ingress of flushing liquid into its interior, and the outlet opening (4a, 4b) in the storage container being arranged such that only active liquid flows out,
• that the outlet opening (4a, 4b) in the storage container is arranged at the bottom when in the use position,
• that a portion of the active liquid is dispensed from the storage container into the flushing liquid during each flushing process,
• that a plate-like distributing element (6) is provided on the holder (2) and comprises a region of action over which flushing liquid flows during the flushing process,
• that the interior of the storage container (3a, 3b) is permanently connected to the distributing element (6) by means of the outlet opening (4a, 4b) by an assembly that prevents the active liquid from flowing freely being arranged therebetween,
• at least one functional chamber (7) for receiving a fragrance-emitting carrier material being provided on the dispensing device (1),
• the functional chamber (7) being designed such that it substantially completely surrounds the carrier material, and
• flushing water cannot flow into the functional chamber (7),
• the functional chamber (7) comprising at least one opening which is closed by a fragrance-permeable membrane (9), such that
• the carrier material is held in the functional chamber (7) and fragrances can be released from the carrier material out of the functional chamber (7) into the surrounding area, **characterized in that**
• the dispensing device (1) comprises at least one means (8) by which flushing water can be guided to the membrane (9) such that the flushing water flows over the membrane (9) each time the flushing water is released and said membrane is wetted.

2. The dispensing device according to claim 1, **characterized in that** the functional chamber (7) comprises an opening (9) which is covered with a membrane and points towards the means (8) by which flushing water is guided into the functional chamber (7).

3. The dispensing device according to one of the preceding claims, **characterized in that** the functional chamber (7) comprises at the top membrane-covered openings (11) and/or openings (12) in the lateral surface which allow fragrance to escape from the functional chamber (7).

4. The dispensing device according to one of the preceding claims, **characterized in that** the functional chamber (7) is formed by a first trough-shaped part (16) and a second part (17) that closes the trough-shaped part (16).

5. The dispensing device according to one of the preceding claims, **characterized in that** the holder (2) and the attachment element (10) are designed such that the functional chamber (7) is positioned outside the flow of flushing water when the dispensing device (1) is in the use position.

6. The dispensing device according to one of the preceding claims, **characterized in that** the carrier material is a fluid.

7. The dispensing device according to one of the preceding claims, **characterized in that** the carrier material has a solid or gel-like physical state.

8. The dispensing device according to one of the preceding claims, **characterized in that** the carrier material is selected from the group of polymers, celluloses, porous materials, sponges, rinsable or non-rinsable gels, water-soluble or insoluble films, alcohols, oils, or aqueous solutions.

9. The dispensing device according to one of the preceding claims, **characterized in that** the means by which the flushing water is guided into the functional chamber (7) is in the form of a scoop-like channel (8) which is open at the top and arranged on the dispensing device such that, when the dispensing device is in the intended use state on the toilet bowl, the channel (8) lies below the flushing water outlet of the toilet bowl and at least some of the flushing water collects in the channel (8) during its outflow from the inner rim of the toilet bowl and is guided therefrom to the membrane (9) of the functional chamber (7).

10. The dispensing device according to claim 9, **characterized in that** the channel (8) is arranged above the plate-like distribution element (6).

11. The dispensing device according to one of claims 9 and 10, **characterized in that** the channel (8) and the plate-like distribution element (6), pointing towards the inner rim of a toilet bowl, protrude into the toilet bowl such that, when in the intended use state, the dispensing device (1) is fixed in the toilet bowl in a substantially vertical position with respect to the axis (M).

12. The dispensing device according to one of claims 9-11, **characterized in that** the channel (8) is fixed to the attachment element (10).

13. The dispensing device according to one of the preceding claims, **characterized in that** at least one additional storage container (3b) for an active liquid is provided in the holder (2), the storage container (3b) comprising an outlet opening (4b) via which the active liquid can be dispensed into the rinsing fluid.

## Revendications

1. Distributeur (1) destiné à délivrer au moins un fluide actif dans l'eau de chasse d'une cuvette de toilettes pour parfumer l'environnement du distributeur (1), comprenant
• un support (2) pouvant être suspendu au bord de la cuvette de toilettes et
• au moins un réservoir (3a, 3b) prévu dans le support (2) au destiné à un fluide actif, le réservoir (3a, 3b) comportant au moins un orifice de sortie (4a, 4b) par lequel le fluide actif peut être délivré dans le liquide de chasse,
• le liquide de chasse étant empêché d'entrer à l'intérieur du réservoir (3a, 3b) et l'orifice de sortie (4a, 4b) du réservoir étant disposé de telle sorte que seule le fluide actif sort,
• de telle sorte que l'orifice de sortie (4a, 4b) du réservoir est disposé du côté fond dans la position d'utilisation,
• de telle sorte, à chaque opération de chasse, la délivrance d'une quantité partielle du fluide actif est effectuée dans le sens allant du réservoir dans le liquide de chasse,
• telle sorte qu'il est prévu au niveau du support (2) un élément de répartition (6) en forme de plaque qui comporte une zone d'impact qui est submergée par le liquide de chasse lors du processus de chasse,
• de telle sorte que l'intérieur du réservoir (3a, 3b) est en liaison permanente avec l'élément de distribution (6) par le biais de l'orifice de sortie (4a, 4b) avec interposition d'un dispositif empêchant l'écoulement libre du fluide actif,
• au moins une chambre fonctionnelle (7) étant prévue pour recevoir un substrat d'émission de parfum au niveau du distributeur (1),
• la chambre fonctionnelle (7) étant configurée de manière à entourer sensiblement complètement le substrat et
• l'eau de chasse ne pouvant pas entrer dans la chambre fonctionnelle (7),
• la chambre fonctionnelle (7) comportant un orifice fermé par une membrane (9) perméable aux parfums,
• le substrat étant retenu dans la chambre fonctionnelle (7) et des parfums pouvant être libérés par le substrat de la chambre fonctionnelle (7) dans l'environnement, **caractérisé en ce que**
• le distributeur (1) comporte au moins un moyen (8) qui permet de guider l'eau de chasse à la membrane (9) de sorte que la membrane (9) est submergée et mouillée par l'eau de chasse à chaque déclenchement de l'écoulement d'eau de chasse.

2. Distributeur selon la revendication 1, **caractérisé en ce que** la chambre fonctionnelle (7) comporte un orifice (9) recouvert par une membrane et dirigé vers le moyen (8) qui permet de guider l'eau de chasse jusque dans la chambre fonctionnelle (7).

3. Distributeur selon l'une des revendications précédentes, **caractérisé en ce que** la chambre fonctionnelle (7) comporte des orifices (11) côté tête recouverts d'une membrane et/ou des orifices (12) ménagés dans la surface d'enveloppe qui permettent au parfum de sortir de la chambre fonctionnelle (7).

4. Distributeur selon l'une des revendications précédentes, **caractérisé en ce que** la chambre fonctionnelle (7) est formée d'une première partie (16) en forme cuvette et d'une seconde partie (17) fermant la partie 16) en forme de cuvette.

5. Distributeur selon l'une des revendications précédentes, **caractérisé en ce que** le support (2) et l'élément de fixation (10) sont configurés de telle sorte que la chambre fonctionnelle (7) est positionnée à l'extérieur de l'écoulement d'eau de chasse dans la position d'utilisation du distributeur (1).

6. Distributeur selon l'une des revendications précédentes, **caractérisé en ce que** le substrat est un liquide.

7. Distributeur selon l'une des revendications précédentes, **caractérisé en ce que** le substrat présente un état physique solide ou gélatineux.

8. Distributeur selon l'une des revendications précédentes, **caractérisé en ce que** le substrat est choisi dans le groupe des polymères, des celluloses, des matériaux poreux, des éponges, des gels lavables ou non lavables, des films solubles ou insolubles dans l'eau, des alcools, des huiles ou des solutions aqueuses.

9. Distributeur selon l'une des revendications précédentes, **caractérisé en ce que** le moyen, qui permet de guider l'eau de chasse jusque dans la chambre fonctionnelle (7), est conformé en canal (8), en forme d'ailette ouverte vers le haut, qui est disposé au niveau du distributeur de telle sorte que, dans l'état d'utilisation normal du distributeur au niveau de la cuvette de toilettes, le canal (8) est situé au-dessous de la sortie d'eau de chasse de la cuvette de toilettes et au moins une partie de l'eau de chasse est collectée à la sortie du bord intérieure de la cuvette de toilettes dans le canal (8) puis est guidée sur la membrane (9) de la chambre fonctionnelle (7).

10. Distributeur selon la revendication 9, **caractérisé en ce que** le canal (8) est situé au-dessus de l'élément de répartition (6) en forme de plaque.

11. Distributeur selon la revendication 9 ou 10, **caractérisé en ce que** le canal (8) et l'élément de répartition (6) en forme de plaque fait saillie dans la cuvette de toilette en étant dirigé vers le bord intérieur de la cuvette de toilette de sorte que, dans l'état d'utilisation normal, le distributeur (1) est fixé dans la cuvette de toilettes à une position sensiblement verticale par rapport à l'axe M.

12. Distributeur selon l'une des revendications 9 à 11, **caractérisé en ce que** le canal (8) est fixé à l'élément de fixation (10).

13. Distributeur selon l'une des revendications précédentes, **caractérisé en ce qu'**au moins un autre réservoir (3b) destiné à un fluide actif est prévu dans le support (2), le réservoir (3b) comportant un orifice de sortie (4b) par lequel le fluide actif peut être délivré dans lé liquide de chasse.
